# EUROPÄISCHE PATENTSCHRIFT

(11) **EP 3 564 664 B1**
(45) Veröffentlichungstag und Bekanntmachung des Hinweises auf die Patenterteilung: **07.04.2021**
(21) Anmeldenummer: 19155764.4
(22) Anmeldetag: 06.02.2019
(51) Int. Cl.: E03B 7/08, C02F 1/00, C02F 1/28, G01N 33/18

(54) **WASSERARMATUR MIT SPÜLAUTOMATIK**
WATER FITTING WITH AUTOMATED FLUSHING
ROBINETTERIE D'EAU POURVU DE SYSTÈME AUTOMATIQUE DE RINÇAGE

(30) Priorität: 04.05.2018 DE 202018102493 U
(43) Veröffentlichungstag der Anmeldung: 06.11.2019
(73) Patentinhaber: Hans Sasserath GmbH & Co. KG., 41352 Korschenbroich (DE)
(72) Erfinder: Hecking, Willi, 41372 Niederkrüchten-Elmpt (DE)
(74) Vertreter: Weisse, Renate

(56) Entgegenhaltungen:
- EP-A1- 2 778 560
- WO-A1-2015/197255
- DE-A1-102015 203 753

## Beschreibung

### Technisches Gebiet

Die Erfindung betrifft eine Wasserarmatur mit Spülautomatik, enthaltend
(a) ein Armaturengehäuse mit einem Einlass zum Anschließen an eine Wasserversorgung;
(b) einen Ablauf zum Auslassen von Spülwasser aus der Wasserarmatur;
(c) eine Absperrung zum Absperren des Ablaufs; und
(d) eine Absperrautomatik mit einer Steuerung zum Steuern der Absperrung und mit Mitteln zum Erzeugen eines Alarmsignals; und
(e) einen Leitfähigkeitssensor, mit dessen Signalen die Absperrautomatik beaufschlagt ist.

Ein Beispiel für eine Wasserarmatur mit einer Spülautomatik ist ein Rückspülfilter. Wasser wird durch den Filter geleitet und dabei von Partikeln gereinigt. Mit der Zeit setzt der Filter sich zu. Bei einem Rückspülfilter kann das Filtermaterial gereinigt werden. Das Wasser fließt dann vom Einlass in umgekehrter Richtung durch das Filtermaterial zu einem Ablauf und wird von dort entsorgt. Dabei werden die Schmutzpartikel vom Filtermaterial mitgerissen.

Ein weiteres Beispiel für eine Wasserarmatur mit einer Spülautomatik ist eine Armatur zur Durchführung einer Hygienespülung. Eine Wasserinstallation ist beispielsweise eine Trinkwasserversorgung in einem Gebäude. Wenn über einen längeren Zeitraum kein Wasser gezapft wird, stagniert das Wasser. Bei diesen Verhältnissen können sich Keime bilden. Das ist ein unerwünschter Strömungszustand. Es ist daher bekannt, eine Armatur mit einer mit einer Absperrung versehenen Verbindung zu einem Ablauf in der Wasserinstallation vorzusehen. Die Absperrung wird regelmäßig geöffnet. Dann wird eine Wassermenge in den Ablauf geleitet, die ausreicht um die Wasserinstallation zu spülen.

Spülungen mit bekannten Anordnungen können durch Öffnen der Absperrung von Hand durchgeführt werden. Komfortablere Lösungen sehen aber eine Absperrautomatik vor. Die Absperrautomatik umfasst eine Steuerung zum Steuern der Absperrung, wenn eine Spülung erforderlich ist. Dann wird die Absperrung für einen begrenzten Zeitraum mittels eines Motors geöffnet und die Spülung durchgeführt. Die Steuerung kann die Spülung nach Ablauf einer vorgegebenen Zeitspanne oder aufgrund eines Messsignals auslösen.

Wasser aus einer Wasserversorgung kann nicht nur Verkeimung und Verunreinigung unterliegen. Es enthält gewöhnlich auch Kalk. Der Kalkgehalt des Wassers wird als Wasserhärte ausgedrückt. Hartes Wasser enthält viel gelöste Kationen, insbesondere Calciumionen. Weiches Wasser enthält wenig gelöste Kationen. Während eine gewisse Menge an Kationen im Wasser der Gesundheit förderlich ist, ist eine zu hohe Wasserhärte unerwünscht. Kalk führt zu unerwünschten Kalkablagerungen in der Wasserinstallation und in angeschlossenen Geräten. Es ist daher bekannt, das Wasser auf ein gewünschtes Niveau zu enthärten.

Typischerweise veröffentlichen die Wasserwerke die Wasserhärte des von ihnen gelieferten Wassers. Es ist auch möglich, die Wasserhärte zu messen. Die Messung oder Überprüfung der Wasserhärte erfolgt insbesondere, wenn ein Gebäude mit den erforderlichen Wasserarmaturen ausgestattet wird. Danach ist eine aufwändige Kontrolle der Wasserhärte unüblich. Eine Änderung der Wasserhärte bleibt daher von den Gebäudebesitzern in der Regel unbemerkt.

### Stand der Technik

WO 2014/040823A1 offenbart eine Wasserarmatur zur Durchführung einer Hygienespülung in einer Wasserinstallation. Wasser aus der Wasserinstallation ist über eine Ablaufanordnung abführbar, so dass Stagnation in der Wasserinstallation vermieden wird. Die Wasserarmatur weist einen Anschluss zum Anschließen der Wasserarmatur an eine Frischwasserversorgung und eine Absperrung zum Unterbrechen der Frischwasserzufuhr aus der Frischwasserversorgung auf. Eine Anordnung zur Überprüfung des Hygienestatus einer Trinkwasserinstallation ist in WO 2015/197255 A1 offenbart.

DE 10 2009 003 343 B4 offenbart einen Rückspülfilter mit integriertem Druckminderer. DE 10 2014 101 285 A1, EP 2 778 560 A1 und DE 10 2015 203 753 A1 offenbaren Anordnungen zur Enthärtung von Trinkwasser, welche Kommunikationsmittel aufweisen, über welche die Enthärtungseinrichtung ansteuerbar ist. Zur Ermittlung der Wasserhärte wird jeweils ein Leitfähigkeitssensor eingesetzt.

### Offenbarung der Erfindung

Es ist Aufgabe der Erfindung, eine kostengünstige Möglichkeit zu schaffen, die Wasserhärte regelmäßig zu überprüfen. Erfindungsgemäß wird die Aufgabe bei einer Wasserarmatur der eingangs genannten Art dadurch gelöst, dass
(e) der Leitfähigkeitssensor im Ablauf vorgesehen ist, um die Leitfähigkeit des Spülwassers zu messen; und
(f) das Alarmsignal bei einer Abweichung der Leitfähigkeit im Spülwasser von einem Sollwertbereich oder einem Sollwert erzeugt wird.

Bei einer erfindungsgemäßen Wasserarmatur wird die Absperrautomatik nicht nur dazu verwendet, eine Spülung auszulösen, sondern gleichzeitig dazu, die Wasserhärte zu überprüfen. Die Leitfähigkeit des Wassers ist nämlich ein vergleichsweise guter Indikator für die Wasserhärte. Ein hoher Ionenanteil im Wasser bewirkt eine hohe Leitfähigkeit. Ein niedriger Ionenanteil im Wasser bewirkt eine niedrige Leitfähigkeit. Die meisten Ionen sind härtebildende Kalziumionen. Die Signale des Leitfähigkeitssensors werden von der Absperrautomatik ausgewertet und es wird ein Alarmsignal erzeugt, wenn die Leitfähigkeit im Spülwasser außerhalb eines Sollwertbereichs liegt oder von einem Sollwert abweicht.

Ein geänderter Härtegrad des Wassers ist für einen begrenzten Zeitraum hinnehmbar. Es ist daher nicht erforderlich, die Leitfähigkeit und damit die Wasserhärte ständig zu messen und zu überprüfen. Eine gelegentliche Überprüfung ist aber insbesondere in den Wasserinstallationen sinnvoll, für die gewöhnlich Wasser mit einer geringen Wasserhärte bereitgestellt wird. In solchen Wasserinstallationen wird keine Wasserenthärtungseinrichtung verwendet. Eine erhöhte Wasserhärte kann dann zu Verkalkung der Installation und Armaturen führen.

Es hat sich herausgestellt, dass die Abstände, in denen eine Spülung, etwa bei einem Filter oder einer Hygienearmatur, durchgeführt wird, gerade ausreichend sind um die Wasserhärte zu ermitteln. Es wird also jedesmal, wenn eine Spülung durchgeführt wird, die Leitfähigkeit gemessen. Die doppelte Verwendung der Absperrautomatik zur Verarbeitung und/oder Übertragung des Leitfähigkeitssignals und zur Steuerung des Spülvorgangs vermeidet die Notwendigkeit einer weiteren elektronischen Platine mit Gehäuse, Schnittstelle und Kommunikationseinrichtung. Dadurch werden trotz zusätzlicher Funktionalität Raum und Resourcen gespart.

Bei einer weiteren Ausgestaltung der Erfindung ist der Leitfähigkeitssensor im Ablauf stromabwärts von der Absperrung angeordnet. Dann wird ein Signal immer nur bei einer Spülung erzeugt. Es ist keine zusätzliche Steuerung erforderlich.

Vorteilhafterweise ist vorgesehen, dass der Ablauf einen Ablaufkanal umfasst, dessen Abmessungen derart bemessen sind, dass die Leitfähigkeit des Spülwassers mit dem Leitfähigkeitssensor bei einer Spülung der Wasserarmatur erfassbar sind. Dabei ist der Leitfähigkeitssensor hinter der Absperrung, d.h. im drucklosen Teil der Armatur angeordnet. Wenn die Absperrung geöffnet ist, fließt in der Regel ein hoher Volumenstrom durch den Ablauf. Die Abmessungen des Ablaufkanals sind so bemessen, dass einerseits alles Wasser gut ablaufen kann und andererseits praktisch keine Luft im Bereich des Leitfähigkeitssensors vorliegt. Zu viel Luft könnte die Richtigkeit der Messergebnisse des Leitfähigkeitssensors beeinträchtigen. Bei geeigneten Abmessungen und insbesondere geeignetem Durchmesser, welcher schmaler ist, als der Durchmesser bekannter Abläufe in bekannten Spülarmaturen, kann die Richtigkeit hingegen erhöht werden.

Die Messung der Leitfähigkeit im drucklosen Teil hinter der Absperrung hat den Vorteil, dass der Leitfähigkeitssensor leicht ausgetauscht oder gewartet werden kann. Es muss keine Absperrung für das Frischwasser vorhanden sein und der wasserführende Teil der Armatur muss nicht geöffnet werden um Zugang zum Leitfähigkeitssensor zu erhalten.

Die Absperrung kann von einem Kugelhahn oder einem Magnetventil gebildet sein. Es ist aber auch möglich, eine andere Absperrung zu verwenden.

Das Alarmsignal kann akustisch oder optisch direkt an der Absperrautomatik erzeugt werden. Es kann aber auch vorgesehen sein, dass die Absperrautomatik eine Signalverarbeitungseinrichtung und eine Kommunikationseinrichtung zur Kommunikation mit weiteren Signalverarbeitungseinrichtungen in der Wasserinstallation oder einem Server aufweist. Das Signal kann über die Kommunikationseinrichtung auch an Mobilfunkendgeräte, einen digitalen Assistenten, eine Techniksteuerung des Gebäudes oder andere Geräte übertragen werden, welche geeignet sind, auf den geänderten Härtegrad des Wassers aufmerksam zu machen.

Bei einer Ausgestaltung der Erfindung umfasst die Wasserarmatur Mittel zur automatischen Durchführung einer Hygienespülung in einer Wasserinstallation. Solche Mittel können eine Turbine oder einen anderen Strömungsmesser umfassen. Mit dem Strömungsmesser wird ermittelt, wieviel Wasser durch die Wasserinstallation fließt. Wenn für einen längeren Zeitraum kein Wasser gezapft oder verbraucht wurde, besteht Stagnationsgefahr. Dabei können sich Keime bilden. Dies wird vermieden, indem die Absperrung geöffnet wird, wenn über einen längeren Zeitraum kein Wasser gezapft oder verbraucht wurde.

Die Wasserarmatur kann aber auch alternativ oder zusätzlich einen Rückspülfilter oder einen Druckminderer-Rückspülfilter umfassen. Der Rückspülfilter wird zum Reinigen regelmäßig rückgespült.

Es kann aber auch jede andere Wasserarmatur verwendet werden, die weitere Komponenten und Funktionalitäten aufweist oder für andere Zwecke vorgesehen ist, solange von Zeit zu Zeit eine Spülung vorgesehen ist.

Wenn festgestellt wird, dass das Wasserwerk Wasser mit einer neuen Wasserhärte bereitstellt, können geeignete Maßnahmen ergriffen werden. Bei einer höheren Wasserhärte können beispielsweise Enthärtungseinrichtungen installiert werden, mit denen das Wasser auf einen gewünschten Härtegrad enthärtet wird.

Erfindungsgemäß wird die Aufgabe bei einer Wasserarmatur der eingangs genannten Art auch dadurch gelöst, dass
(d) der Ablauf einen Ablaufkanal mit einer lateralen Bohrung umfasst;
(e) die laterale Bohrung mit einem lösbaren Stopfen verschlossen ist; und
(f) die Bohrung derart angeordnet ist, dass die Wasserarmatur mit einer Absperrautomatik mit einer Steuerung zum Steuern der Absperrung nachrüstbar ist, welche einen Leitfähigkeitssensor zum Messen der Leitfähigkeit des Spülwassers aufweist, der in die laterale Bohrung einführbar ist.

Insbesondere kann vorgesehen sein, dass alle Filter einen Ablaufkanal verwenden, welcher eine mit einem Stopfen verschlossene, laterale Bohrung zum Nachrüsten mit einem Leitfähigkeitssensor aufweist. Dann kann der Filter einerseits handbetätigt mit einem Griff und mit dem Stopfen verschlossener Bohrung ausgebildet sein. Ein solcher Filter kann leicht nachgerüstet werden. Der Griff und der Stopfen liegen im drucklosen Bereich und können bei geschlossenem Kugelhahn leicht entfernt werden. An ihrer Stelle wird die Absperrautomatik mit dem Leitfähigkeitssensor installiert.

Ausgestaltungen der Erfindung sind Gegenstand der Unteransprüche. Ein Ausführungsbeispiel ist nachstehend unter Bezugnahme auf die beigefügten Zeichnungen näher erläutert.

### Definitionen

In dieser Beschreibung und in den beigefügten Ansprüchen haben alle Begriffe eine dem Fachmann geläufige Bedeutung, welche der Fachliteratur, Normen insbesondere DIN EN 806-1 und DIN EN 1717 und den einschlägigen Internetseiten und Publikationen, insbesondere lexikalischer Art, beispielsweise www.Wikipedia.de, www.wissen.de oder www.techniklexikon.net, der Wettbewerber, forschenden Institute, Universitäten und Verbände, beispielsweise Deutscher Verein des Gas- und Wasserfaches e.V. oder Verein Deutscher Ingenieure, dargelegt sind. Insbesondere haben die verwendeten Begriffe nicht die gegenteilige Bedeutung dessen, was der Fachmann den obigen Publikationen entnimmt.

Weiterhin werden hier folgende Bedeutungen für die verwendeten Begriffe zugrunde gelegt:
- Absperrung:: ist jede Art von Einrichtung, welche einen Fluidstrom ganz oder teilweise blockiert. Typische Absperrungen sind Magnetventile, Kugelhähne oder Ventile.
- Armatur:: ist ein Bauteil zur Installation in oder an einer Rohrleitung oder anderen Fluidinstallation zum Absperren, Regeln oder Beeinflussen von Stoffströmen. Eine Armatur kann einteilig oder mehrteilig ausgebildet sein und wird an einer Stelle in oder an der Rohrleitung installiert. Armaturen sind beispielsweise und nicht abschließend: Anschlussvorrichtungen, Anschlussarmaturen, Hauptabsperrarmaturen, Wartungsarmaturen, Drosselarmaturen, Entnahmestellen, Entnahmearmaturen, Entleerungsarmaturen, Sicherungsarmaturen, Sicherheitsarmaturen und Stellarmaturen.
- axial: ist die Richtung der Rotationsachse von ganz oder teilweise rotationssymmetrischen Bauteilen, wie etwas Rohren oder langgestreckten Gehäusen. Bei Bauteilen ohne Rotationssymmetrie ist es die Hauptströmungsrichtung in einem Bauteilabschnitt.
- Bohrung: ist jede Art von Verbindung zweier Hohlräume, sowie Sacklöcher.
- Druck: Kraft pro Flächeneinheit
- Druckminderer: ist eine Armatur zur Einstellung eines ausgewählten Drucks an dahinterliegenden Bauteilen.
- Einlass: ist eine zulaufseitige Öffnung in einem Gehäuse, in welchen ein Stoffstrom hineinfließen kann. Die Öffnung kann insbesondere an eine Rohrleitung oder eine weitere Armatur angeschlossen sein oder frei zur Atmosphäre hin öffnen.
- Einlassanschluss: Einlass, der den Anschluss von Leitungen, Schläuchen, Rohren oder weiteren Armaturen erlaubt.
- Gehäuse: Begrenzung für Stoffe, Bauteile, Instrumente und Messgeräte nach außen. Ein Gehäuse kann einteilig oder aus mehreren verbundenen Gehäuseteilen mehrteilig ausgebildet sein und aus einem oder mehreren Materialien bestehen.
- Mutter: Maschinenelement zur Herstellung lösbarer Verbindungen. Die Mutter ist ein Hohlkörper mit Innengewinde.
- radial: senkrecht zu einer axialen Richtung.
- Rohr: Hohlkörper aus zylindrischen Abschnitten. Dient üblicherweise als Rohrleitung.
- Schulter: Übergang von Abschnitten unterschiedlicher Durchmesser oder Dicken.
- Sensor: technisches Bauteil, das physikalische oder chemische Eigenschaften und/oder die stoffliche Beschaffenheit seiner Umgebung qualitativ und/oder quantitativ erfassen kann. Beispiele sind Strömungsmesser, Temperatursensoren und Drucksensoren, aber auch Leitfähigkeitssensoren und pH-Messer.
- Stutzen: Rand oder Übergangsstück an einer Öffnung.
- Turbine: rotierende Strömungsmaschine, beispielsweise ein Flügelrad, welche das Abfallen der inneren Energie eines strömenden Fluides in mechanische Leistung umwandelt, die sie über ihre Welle abgibt.
- Ventil: Bauteil zur Absperrung oder Regelung des Durchflusses von Fluiden.
- zapfen: entnehmen von Wasser aus einer Installation, beispielsweise an einem Wasserhahn oder mittels eines Apparates.
- Zapfstelle: Anordnung zum zapfen. Beispiele sind Waschbecken, Spülbecken, Duschen, Badewannen, Bidets, Toiletten mit den zugehörigen Hähnen bzw. Zapfarmaturen.
- Wasserinstallation: wasserführende oder Wasser enthaltende Installationen. Sie können Rohre, Schläuche, Container, Armaturen, Maschinen und dergleichen enthalten. Beispiele sind die Wasserversorgung in Gebäuden, Schwimmbäder, Saunen, Aquarien, künstliche Teiche, Bewässerungsanlagen und Brunnen oder industrielle Anwendungen. Die Wasserinstallation kann in mehrere Stränge aufgeteilt sein.
- Wasserverbraucher: Zapfstelle, an welcher ein Apparat oder eine Maschine angeschlossen ist. Beispiele sind Waschmaschinen oder Spülmaschinen.

### Kurze Beschreibung der Zeichnungen

- Fig.1: ist eine perspektivische Darstellung einer Wasserarmatur mit einem Rückspülfilter und einer Rückspülautomatik.
- Fig.2: zeigt eine handbetätigte Wasserarmatur ähnlich wie in Figur 1, aber mit Stellgriff und ohne Rückspülautomatik als Explosionsdarstellung.
- Fig.3: zeigt die Wasserarmatur aus Figur 1 mit Rückspülautomatik als Explosionsdarstellung.
- Fig.4: ist ein Vertikalschnitt durch die Wasserarmatur aus Figur 1.
- Fig.5: ist ein Vertikalschnitt durch einen Teil einer Wasserarmatur aus Figur 2 und zeigt den Ablauf im Detail.

### Beschreibung des Ausführungsbeispiels

Die Figuren zeigen eine allgemein mit 10 bezeichnete Wasserarmatur. Die Wasserarmatur weist ein Gehäuse mit einem oberen Gehäuseteil 12 und einer Filtertasse 14 auf. Der obere Gehäuseteil 12 ist mit einem Flansch 16 an einen Flansch einer korrespondierenden Anschlussarmatur (nicht dargestellt) angeflanscht. Es versteht sich, dass statt eines Flanschanschlusses auch eine Installation direkt in der Rohrleitung verwendet werden kann. Derartige Flanschanschlüsse sind allgemein bekannt und brauchen daher hier nicht näher erläutert werden. Der Einlass wird hier von einem Zentralkanal gebildet und der Auslass von einem darum herum angeordneten Ringkanal oder umgekehrt.

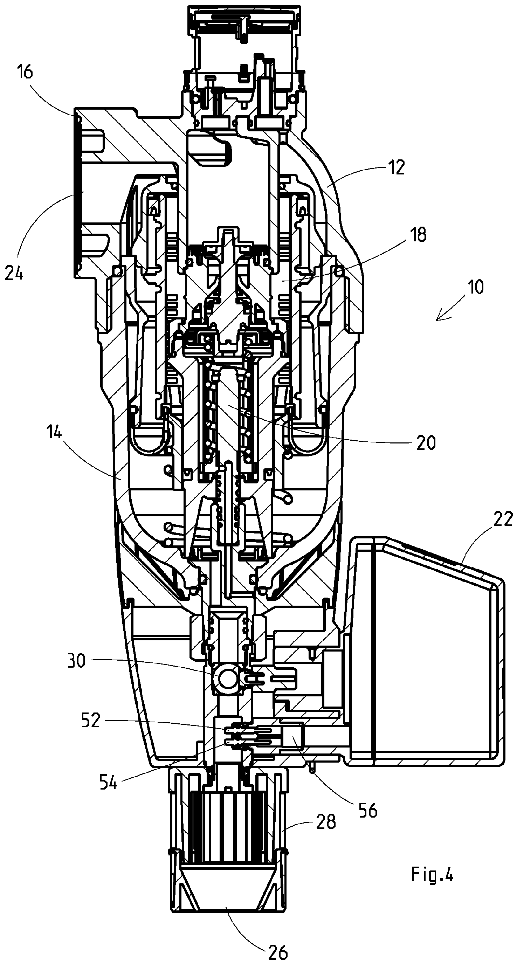

Wasser fließt in das Gehäuse und wird dort in einem allgemein mit 18 bezeichneten Filter gefiltert. Es fließt anschließend vom Gehäuse zurück zum Anschlussflansch 16 und zurück in die Rohrleitung. Im vorliegenden Ausführungsbeispiel ist zusätzlich ein Druckminderer 20 vorgesehen. Dieser ist in Figur 4 gut zu erkennen. Filter und Druckminderer-Filterkombinationen der verwendeten Art sind allgemein bekannt und brauchen hier daher nicht näher erläutert werden. Wichtig für die vorliegende Erfindung ist es, dass der Filter 18 ein Rückspülfilter ist.

Schmutzpartikel bleiben an dem Filtermaterial haften. Mit der Zeit setzt sich der Filter 18 zu. Der vorliegende Filter ist ein Rückspülfilter 18, der durch Rückspülen gereinigt wird. Eine Rückspülung wird entweder in regelmäßigen zeitlichen Abständen ausgelöst oder der Druckabfall an dem Filter wird gemessen. Bei Erreichen eines vorgegebenen Druckabfalls oder wenn ein vorgegebenes Zeitintervall erreicht ist, wird eine Rückspülung ausgelöst.

Bei der Rückspülung wird die Strömung durch den Filter 18 umgekehrt. Wasser fließt vom Einlass 24 in umgekehrter Richtung durch den Filter 18 zu einem Ablauf 26. Der Ablauf 26 ist mit einer Absperrung - hier in Form eines Kugelhahns 30 - versehen. Zum Rückspülen wird der Kugelhahn 30 geöffnet. In einer Betriebsstellung ist der Kugelhahn 30 geschlossen. Das Spülwasser reißt die Schmutzpartikel vom Filter 18 mit. Dadurch wird der Filter gereinigt. Der Ablauf 26 ist mit einem Ablauftrichter 28 versehen oder mit einer Abwasserrohrleitung verbunden.

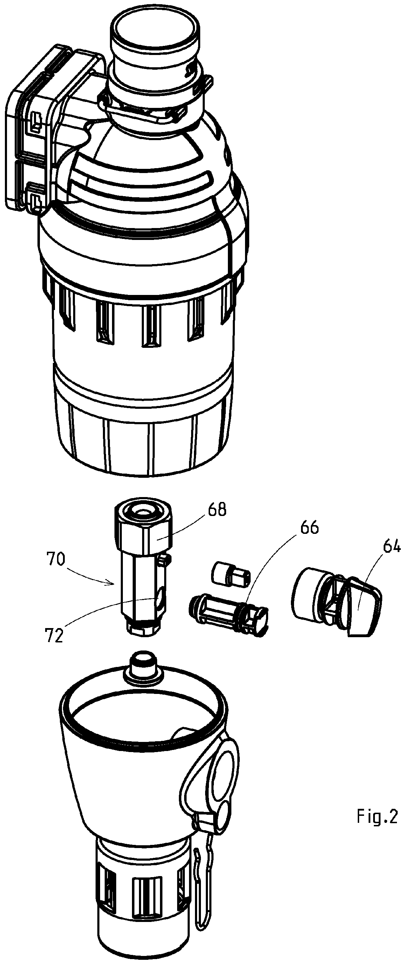

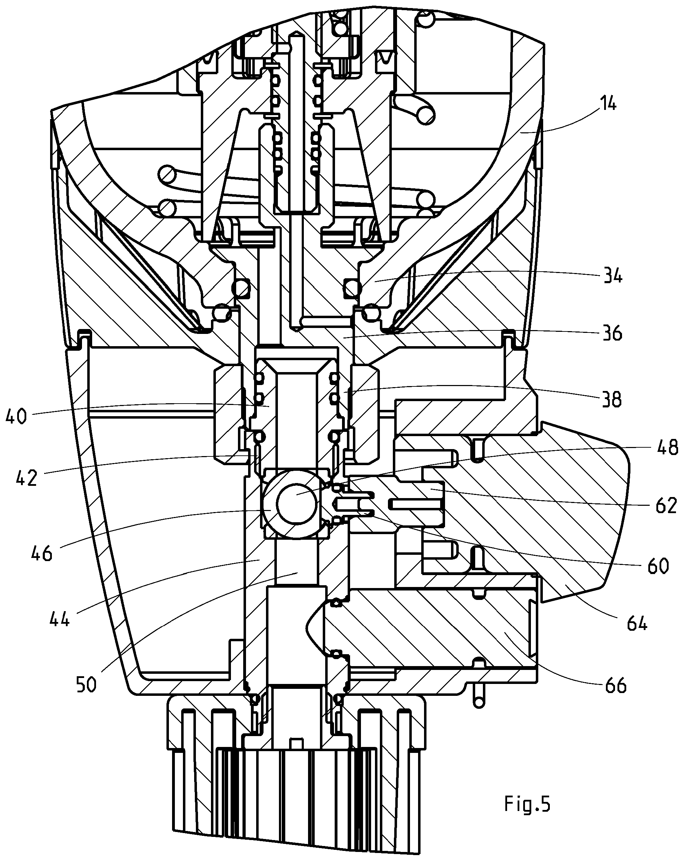

Die Rückspülung des Filters 18 kann mittels eines Handgriffs 64 von Hand ausgelöst werden. Dies ist in Figur 2 und Figur 5 illustriert. Der Handgriff 64 und damit die Kugel des Kugelhahns 30 wird von Hand gedreht und nach einer Zeit, bei der anzunehmen ist, dass die Rückspülung ausreichend ist, wieder zurück gedreht. Ein solcher Rückspülfilter ist vergleichsweise kostengünstig.

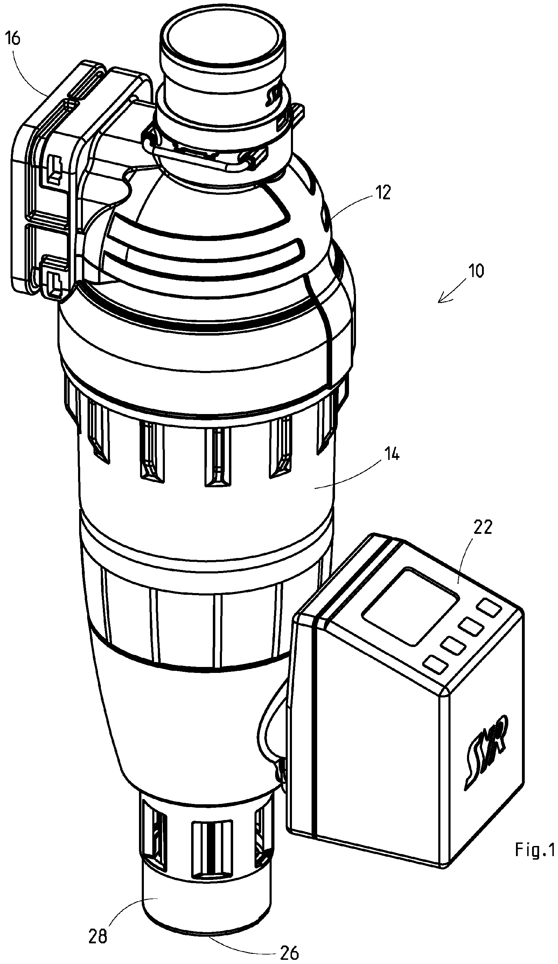

Zum Auslösen und Beenden der Rückspülung ist bei einem alternativen Ausführungsbeispiel eine Rückspülautomatik 22 vorgesehen. Dies ist in Figur 1, 3 und 4 illustriert. Die Rückspülautomatik 22 weist eine Datenverarbeitungseinrichtung, ggf. einen Zeitgeber und eine Steuerung zum Erzeugen eines Steuersignals auf. Es ist ferner ein mit dem Steuersignal beaufschlagter Motor vorgesehen. Mit dem Motor wird die Absperrung 30 geöffnet oder geschlossen.

Figur 4 und 5 zeigen den Ablauf 26 mit dem Kugelhahn im Detail. Die Anordnung ist bis auf die nachstehend beschriebenen Unterschiede für handbetätigte und automatisch betätigte Filter gleich. Die Filtertasse 14 bildet eine Öffnung im Boden 34 der Filtertasse. Ein Einsatz 36 mit Dichtungen ist durch die Öffnung im Boden 34 geführt. Der Einsatz 36 weist einen nach unten ragenden Stutzen 38 auf. In den Stutzen 38 ist ein rohrförmiges, oberes Ablaufteil 40 eingesetzt. Das obere Ablaufteil 40 ist am unteren Ende mit einem Gewinde 42 versehen. Auf das Gewinde 42 ist ein unteres Ablaufteil 44 aufgeschraubt. Die Kugel 46 eines Kugelhahns 48 ist im oberen Bereich des unteren Ablaufteils 44 aufgenommen. Das untere Ende des unteren Ablaufteils 44 mündet in dem Ablauftrichter 28.

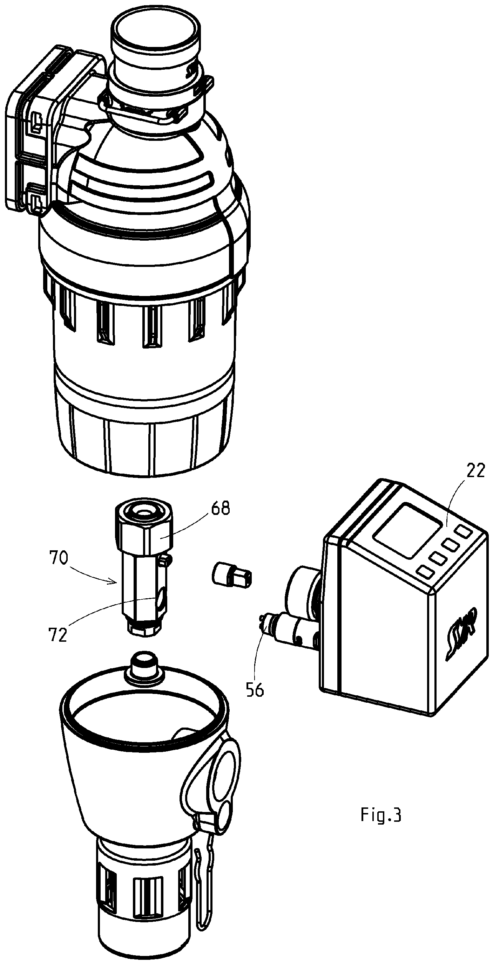

Der Stutzen 38, das obere Ablaufteil 40 und das untere Ablaufteil 44 weisen koaxiale Bohrungen auf, die einen von dem Kugelhahn 48 kontrollierten Ablaufkanal 50 bilden. Die Ablaufbaugruppe 70 aus oberem und unterem Ablaufteil, Kugel und einer Mutter 68 ist in Figur 2 und Figur 3 zusammengesetzt gezeigt.

In Figur 5 ist der Kugelhahn 48 in einer geschlossenen Stellung gezeigt. Dann ist die Wasserarmatur in der Betriebsstellung. Die Kugel 46 des Kugelhahns 48 ist über einen Vierkant mit einem Betätigungselement 60 verbunden. In das Betätigungselement 60 greift ein Zapfen 62. Der Zapfen 62 ist bei der Verwendung einer Rückspülautomatik 22 mit dem Motor der Rückspülautomatik 22 verbunden. Dies ist in Figur 4 dargestellt. Zum Öffnen und Schließen erhält der Motor von der Steuerung der Rückspülautomatik 22 ein Steuersignal. Dann wird der Zapfen 62 und das Betätigungselement 60 um eine in der Darstellungsebene der Figuren liegende Achse gedreht. Dabei wird die Kugel 46 des Kugelhahns 48 in die gewünschte Stellung gedreht. Bei der handbetätigten Variante ist der Zapfen 62 mit einem Griff 64 verbunden und wird von Hand betätigt.

Wenn der Kugelhahn 48 geöffnet wird, strömt das Rückspülwasser durch den Ablaufkanal 50. Der Ablaufkanal 50 ist so dimensioniert, dass sich im Ablaufkanal 50 hinter dem Kugelhahn 48 praktisch keine Luft in das Wasser mischt.

Unterhalb des Kugelhahns 30 ist das untere Ablaufteil 44 mit einer lateralen Bohrung 72 versehen. Diese ist in Figur 2 und Figur 3 gut zu erkennen. Bei der handbetätigten Variante des Rückspülfilters ist die Bohrung 72 mit einem Stopfen 66 verschlossen. Dies ermöglicht das einfache Nachrüsten der Anordnung mit der nachstehend beschriebenen Rückspülautomatik mit Leitfähigkeitsmessung. Hierzu muss lediglich bei geschlossenem Kugelhahn 30 der Griff und der Stopfen 66 entfernt werden.

Im drucklosen Teil des Ablaufkanals 50 hinter dem Kugelhahn 48 sind bei der Variante mit Absperrautomatik die Elektroden 52 und 54 eines Leitfähigkeitssensors 56 angeordnet. Mit dem Leitfähigkeitssensor 56 wird ein Leitfähigkeitssignal erzeugt. Das Leitfähigkeits signal wird an die Rückspülautomatik 22 übertragen. Die Datenverarbeitungseinrichtung der Rückspülautomatik 22 wertet das Leitfähigkeitssignal aus. Wenn die aus der Leitfähigkeit ermittelte Wasserhärte einen Sollwert überschreitet, wird ein Alarmsignal erzeugt. Das Alarmsignal wird von der Kommunikationseinrichtung der Rückspülautomatik 22 zum Beispiel über WLAN an einen Server übertragen. Der Server wird beispielsweise von einem Hersteller betrieben. Über den Server erhält der Eigentümer der Rückspülautomatik oder eine andere geeignete Person eine elektronische Nachricht über einen Nachrichtendienst, SMS, Email oder dergleichen. Der Eigentümer hat dann die Möglichkeit geeignete Maßnahmen zu ergreifen. Beispielsweise kann eine Enthärtungseinrichtung in der Wasserinstallation installiert werden oder es können Entkalkertabletten für Waschmaschinen und Spülmaschinen verwendet werden. Es versteht sich, dass das Alarmsignal auch auf jedem anderen Übertragungsweg, direkt oder indirekt, elektronisch, akustisch oder optisch mitgeteilt werden kann. Insbesondere kann das Signal auch mittels Mobilfunkeinheit direkt an ein mobiles Endgerät des Nutzers übertragen werden.

Im vorliegenden Ausführungsbeispiel wurde eine Druckminderer-Filter-Kombination verwendet, die typischerweise im Hauseingangsbereich der Wasserinstallation installiert ist. Es ist aber auch möglich diese oder eine andere Wasserarmatur zu verwenden, die an einer anderen Stelle in der Wasserarmatur, insbesondere am Strangende verwendet wird. Ein Beispiel für eine solche Wasserarmatur ist eine Einrichtung zur Durchführung einer Hygienespülung. Auch hier wird Wasser über eine Absperrautomatik kontrolliert nach außen abgeführt.

Die oben erläuterten Ausführungsbeispiele dienen der Illustration der in den Ansprüchen beanspruchten Erfindung. Merkmale, welche gemeinsam mit anderen Merkmalen offenbart sind, können in der Regel auch alleine oder in Kombination mit anderen Merkmalen, die im Text oder in den Zeichnungen explizit oder implizit in den Ausführungsbeispielen offenbart sind, verwendet werden. Maße und Größen sind nur beispielhaft angegeben. Dem Fachmann ergeben sich geeignete Bereiche aus seinem Fachwissen und brauchen hier daher nicht näher erläutert werden. Die Offenbarung einer konkreten Ausgestaltung eines Merkmals bedeutet nicht, dass die Erfindung auf diese konkrete Ausgestaltung beschränkt werden soll. Vielmehr kann ein solches Merkmal durch eine Vielzahl anderer, dem Fachmann geläufigen Ausgestaltungen verwirklicht werden. Die Erfindung kann daher nicht nur in Form der erläuterten Ausgestaltungen verwirklicht werden, sondern durch alle Ausgestaltungen, welche vom Schutzbereich der beigefügten Ansprüche abgedeckt sind. Insbesondere können auch mehr oder weniger Sensoren vorgesehen sein. Stutzen, Rohre, Leitungen und Schläuche können einteilig oder mehrteilig ausgebildet sein und etwa Stutzenverlängerungen aufweisen.

Die Begriffe "oben", "unten", "rechts" und "links" beziehen sich ausschließlich auf die beigefügten Zeichnungen. Es versteht sich, dass beanspruchte Vorrichtungen auch eine andere Orientierung annehmen können. Der Begriff "enthaltend" und der Begriff "umfassend" bedeuten, dass weitere, nicht-genannte Komponenten vorgesehen sein können. Unter dem Begriff "im Wesentlichen", "vorwiegend" und "überwiegend" fallen alle Merkmale, die eine Eigenschaft oder einen Gehalt mehrheitlich, d.h. mehr als alle anderen genannten Komponenten oder Eigenschaften des Merkmals aufweisen, also bei zwei Komponenten beispielsweise mehr als 50%.

## Patentansprüche

1. Wasserarmatur (10) mit Spülautomatik, enthaltend
(a) ein Armaturengehäuse mit einem Einlass (24) zum Anschließen an eine Wasserversorgung;
(b) einen Ablauf (26) zum Auslassen von Spülwasser aus der Wasserarmatur;
(c) eine Absperrung (30) zum Absperren des Ablaufs; und
(d) eine Absperrautomatik (22) mit einer Steuerung zum Steuern der Absperrung und mit Mitteln zum Erzeugen eines Alarmsignals; und
(e) einen Leitfähigkeitssensor (56), mit dessen Signalen die Absperrautomatik beaufschlagt ist;
**dadurch gekennzeichnet, dass**
(f) der Leitfähigkeitssensor (56) im Ablauf vorgesehen ist, um die Leitfähigkeit des Spülwassers zu messen; und
(g) das Alarmsignal bei einer Abweichung der Leitfähigkeit im Spülwasser von einem Sollwertbereich oder einem Sollwert erzeugt wird.

2. Wasserarmatur nach Anspruch 1, **dadurch gekennzeichnet, dass** der Leitfähigkeitssensor (56) im Ablauf (26) stromabwärts von der Absperrung (30) angeordnet ist.

3. Wasserarmatur nach Anspruch 2, **dadurch gekennzeichnet, dass** der Ablauf (26) einen Ablaufkanal umfasst, dessen Abmessungen derart bemessen sind, dass die Leitfähigkeit des Spülwassers mit dem Leitfähigkeitssensor (56) bei einer Spülung der Wasserarmatur erfassbar sind.

4. Wasserarmatur nach einem der vorgehenden Ansprüche, **dadurch gekennzeichnet, dass** die Absperrung (30) von einem Kugelhahn oder einem Magnetventil gebildet ist.

5. Wasserarmatur nach einem der vorgehenden Ansprüche, **dadurch gekennzeichnet, dass** die Absperrautomatik (22) eine Signalverarbeitungseinrichtung und eine Kommunikationseinrichtung zur Kommunikation mit weiteren Signalverarbeitungseinrichtungen in der Wasserinstallation oder einem Server aufweist.

6. Wasserarmatur nach einem der vorgehenden Ansprüche, **gekennzeichnet durch** Mittel zur automatischen Durchführung einer Hygienespülung in einer Wasserinstallation.

7. Wasserarmatur nach einem der vorgehenden Ansprüche, **gekennzeichnet durch** einen Rückspülfilter (18).

8. Wasserarmatur (10) mit Spülautomatik, enthaltend
(a) ein Armaturengehäuse mit einem Einlass (24) zum Anschließen an eine Wasserversorgung;
(b) einen Ablauf (26) zum Auslassen von Spülwasser aus der Wasserarmatur; und
(c) eine handbetätigbare Absperrung (48) zum Absperren des Ablaufs;
**dadurch gekennzeichnet, dass**
(d) der Ablauf einen Ablaufkanal mit einer lateralen Bohrung (72) umfasst;
(e) die laterale Bohrung (72) mit einem lösbaren Stopfen (66) verschlossen ist; und
(f) die Bohrung (72) derart angeordnet ist, dass die Wasserarmatur mit einer Absperrautomatik (22) mit einer Steuerung zum Steuern der Absperrung nachrüstbar ist, welche einen Leitfähigkeitssensor (56) zum Messen der Leitfähigkeit des Spülwassers aufweist, der in die laterale Bohrung einführbar ist.

## Claims

1. Water fitting (10) with flushing automatic, comprising
(a) a fitting housing with an inlet (24) for connecting to a water supply;
(b) a drain outlet (26) for draining flushing water from the water fitting;
(c) a shut-off valve (30) for shutting off the drain outlet; and
(d) a shut-off automatic (22) with a control for controlling the shut-off valve and means for generating an alarm signal; and
(e) a conductivity sensor (56) generating signals which are fed to the shut-off automatic;
**characterized in that**
(f) the conductivity sensor (56) is provided in the drain outlet for measuring the conductivity of the flushing water; and
(g) the alarm signal is generated upon deviation of the conductivity of the flushing water from a set value range or a set value.

2. Water fitting according to claim 1, **characterized in that** the conductivity sensor (56) is provided in the drain outlet (26) downstream of the shut-off valve (30).

3. Water fitting according to claim 2, **characterized in that** the drain outlet (26) comprises a drain channel having diameters which are selected such that the conductivity of the flushing water can be detected with the conductivity sensor (56) during a flushing of the water fitting.

4. Water fitting according to any of the preceding claims, **characterized in that** the shut-off valve (30) is a ball valve or a magnet valve.

5. Water fitting according to any of the preceding claims, **characterized in that** the shut-off automatic (22) comprises a signal processing device and a communication device for communicating with further signal processing devices in the water installation or with a server.

6. Water fitting according to any of the preceding claims, **characterized by** means for automatically carrying out a hygiene flushing in a water installation.

7. Water fitting according to any of the preceding claims, **characterized by** a backflushing filter (18).

8. Water fitting (10) with flushing automatic, comprising
(a) a fitting housing with an inlet (24) for connecting to a water supply;
(b) a drain outlet (26) for draining flushing water from the water fitting;
(c) a manually operated shut-off valve (30) for shutting off the drain outlet;
**characterized in that**
(d) the drain outlet comprises a drain channel with a lateral bore hole (72);
(e) the lateral bore hole (72) is closed by a releasable plug (66); and
(f) the bore hole (72) is arranged such that the water fitting can be upgraded with a shut-off automatic (22) having a control for controlling the shut-off valve which is provided with a conductivity sensor (56) for measuring the conductivity of the flushing water which can be inserted into the lateral bore hole.

## Revendications

1. Pièce de robinetterie pour l'eau (10) munie d'un dispositif de lavage automatique, comprenant
(a) un boîtier de pièce de robinetterie muni d'une entrée (24) destinée à être raccordée à un dispositif d'alimentation en eau ;
(b) un dispositif d'écoulement (26) destiné à évacuer l'eau de lavage hors de la pièce de robinetterie pour l'eau ;
(c) un dispositif de fermeture (30) destiné à fermer le dispositif d'écoulement ; et
(d) un dispositif de fermeture automatique (22) muni d'un dispositif de commande destiné à commander le dispositif de fermeture et muni de moyens destinés à générer un signal d'alerte ; et
(e) un capteur de conductivité (56) dont les signaux sont appliqués au dispositif de fermeture automatique ;
**caractérisée en ce que**
(f) le capteur de conductivité (56) est prévu à l'intérieur du dispositif d'écoulement afin de mesurer la conductivité de l'eau de lavage ;
et
(g) le signal d'alerte est généré dans le cas d'un écart de la conductivité de l'eau de lavage par rapport à une plage de valeur de consigne ou à une valeur de consigne.

2. Pièce de robinetterie pour l'eau selon la revendication 1, **caractérisée en ce que** le capteur de conductivité (56) est disposé à l'intérieur du dispositif d'écoulement (26) en aval du dispositif de fermeture (30).

3. Pièce de robinetterie pour l'eau selon la revendication 2, **caractérisée en ce que** le dispositif d'écoulement (26) comprend un canal d'écoulement dont les dimensions sont définies de sorte que la conductivité de l'eau de lavage peut être détectée à l'aide du capteur de conductivité (56) lors d'un lavage de la pièce de robinetterie pour l'eau.

4. Pièce de robinetterie pour l'eau selon l'une quelconque des revendications précédentes, **caractérisée en ce que** le dispositif de fermeture (30) est formé d'un robinet à boisseau sphérique ou d'une électrovanne.

5. Pièce de robinetterie pour l'eau selon l'une quelconque des revendications précédentes, **caractérisée en ce que** le dispositif de fermeture automatique (22) présente une installation de traitement de signaux et une installation de communication destinée à la communication avec d'autres installations de traitement de signaux situées dans l'installation d'eau ou un serveur.

6. Pièce de robinetterie pour l'eau selon l'une quelconque des revendications précédentes, **caractérisée par** des moyens destinés à la réalisation automatique d'un lavage hygiénique dans l'installation d'eau.

7. Pièce de robinetterie pour l'eau selon l'une quelconque des revendications précédentes, **caractérisée par** un filtre de lavage à contre-courant (18).

8. Pièce de robinetterie pour l'eau (10) munie d'un dispositif de lavage automatique, comprenant
(a) un boîtier de pièce de robinetterie muni d'une entrée (24) destinée à être raccordée à un dispositif d'alimentation en eau ;
(b) un dispositif d'écoulement (26) destiné à évacuer l'eau de lavage hors de la pièce de robinetterie pour l'eau ; et
(c) un dispositif de fermeture (48) pouvant être actionné manuellement et destiné à fermer le dispositif d'écoulement ;
**caractérisée en ce que**
(d) le dispositif d'écoulement comprend un canal d'écoulement muni d'un perçage latéral (72) ;
(e) le perçage latéral (72) est fermé hermétiquement par un bouchon (66) pouvant s'enlever ; et
(f) le perçage (72) est disposé de sorte que la pièce de robinetterie pour l'eau munie d'un dispositif de fermeture automatique (22) peut être équipée ultérieurement d'un dispositif de commande destiné à commander le dispositif de fermeture qui présente un capteur de conductivité (56) destiné à mesurer la conductivité de l'eau de lavage et pouvant être introduit à l'intérieur du perçage latéral.
